Europäisches Patentamt

(19) European Patent Office

Office européen des brevets

(11) Numéro de publication: **0 076 762**
**B1**

(12) **FASCICULE DE BREVET EUROPEEN**

(45) Date de publication du fascicule du brevet:
27.12.84

(51) Int. Cl.³: **A 61 K 35/78**

(21) Numéro de dépôt: **82401813.9**

(22) Date de dépôt: **05.10.82**

(54) Composition pharmaceutique à base d'une fraction extraite de mandassi (cyperus articulatus L.).

(30) Priorité: **07.10.81 FR 8118891**

(43) Date de publication de la demande:
**13.04.83 Bulletin 83/15**

(45) Mention de la délivrance du brevet:
**27.12.84 Bulletin 84/52**

(84) Etats contractants désignés:
**AT BE CH DE FR GB IT LI LU NL SE**

(56) Documents cités:
**BIOLOGICAL ABSTRACTS, vol. 66, 1er octobre 1978,
page 3791, no. 38725; P. DENNY et al.: "The importance
of the littoral epiphyton as food for commerical fish in the
recent African man-made lake, Nyumba ya Mungu
reservoir Tanzania"**

(73) Titulaire: **SYNTHELABO, 58, rue de la Glacière,
F-75621 Paris Cedex 13 (FR)**

(72) Inventeur: **Mahaliet, Jean Blaise, 7, rue Baudelaire,
F-93140 Bondy (FR)**
Inventeur: **Gabriel, Jean Pierre, 18, rue Henri IV,
F-37100 Tours St Symphorien (FR)**

(74) Mandataire: **Thouret-Lemaitre, Elisabeth et al, Service
Brevets - SYNTHELABO 58, rue de la Glacière,
F-75621 Paris Cedex 13 (FR)**

# Description

La présente invention concerne l'application en thérapeutique d'une fraction extraite de Mandassi (*Cyperus articulatus* L.) et le procédé d'extraction de cette fraction.

Le Mandassi, ou *Cyperus articulatus* L., est une plante à souche souterraine odoriférante et tige aérienne florifère cylindrique qui pousse en Afrique, en particulier dans les pays suivants: Mauritanie, Mali, Sénégal, Guinée, Liberia, Côte-d'Ivoire, Congo, Haute-Volta, Ghana, Togo, Nigeria et Cameroun.

La titulaire a trouvé que, à partir des racines de Mandassi, il était possible d'extraire une fraction active dans le domaine du système nerveux central, en particulier comme anticonvulsivant.

Selon l'invention, on extrait la fraction active des racines de Mandassi de la manière suivante:

On broie 500 g de rhizomes secs de Mandassi d'origine camerounaise et on les épuise par une décoction de 2 h, avec 10 fois leur poids d'eau.

Le soluté est soutiré et filtré à chaud, il est ensuite concentré sous vide pour le ramener au poids de la plante mise en œuvre.

On verse alors, tout en agitant, 2 fois le poids en volume d'alcool à 95° GL, soit 1 l.

On laisse reposer le mélange réactionnel, puis on isole le précipité sur un filtre Buchner. On reprend ce précipité essoré, non séché par de l'alcool à 70° GL, jusqu'à absence de coloration de la phase alcoolique. Il faut environ dix reprises de 100 ml chacune.

Les phases alcooliques sont alors concentrées sous vide jusqu'à absence de distillation.

Le résidu est repris par 2 fois 100 ml d'alcool à 70° GL avec un essorage entre chaque reprise.

L'insoluble est séché sous vide et constitue la fraction active. On obtient 5,5 g de fraction active.

Cette fraction active se présente sous la forme d'une poudre blanc-gris, soluble dans l'eau et dans l'alcool à 65° GL.

L'activité anticonvulsivante de la fraction ainsi extraite a été étudiée vis-à-vis des convulsions induites par le Cardiazol.

Le protocole expérimental est le suivant:

Les souris reçoivent la solution de produit à tester ou le solvant par voie intrapéritonéale 30 min avant l'injection de l'agent convulsivant et à raison de 20 ml/kg. Le solvant est du Tween 80 à 1% dans de l'eau distillée.

Le Cardiazol est injecté par voie intraveineuse à la dose de 35 mg/kg dans du sérum physiologique à raison de 10 ml/kg.

Les animaux sont ensuite mis dans des cages individuelles en Makrolon et observés pendant 1 h.

Pour chaque lot de 10 souris, on relève le pourcentage de souris présentant des convulsions chroniques, puis on calcule le pourcentage de protection par rapport au lot témoin.

La fraction extraite de Mandassi est mise en solution à diverses concentrations dans le solvant.

Dans le tableau sont donnés les résultats exprimés en pourcentage de protection.

*Tableau*

| Fraction en solution (g/kg) | % de protection |
|---|---|
| 0,315 | 86 |
| 0,158 | 88 |
| 0,079 | 77 |
| 0,039 | 29 |
| 0,017 | 29 |

Les résultats montrent que la fraction de Mandassi extraite selon le procédé de l'invention est active comme anticonvulsivant et peut être utilisée pour le traitement des épilepsies.

L'invention comprend également les compositions pharmaceutiques renfermant la fraction active extraite de Mandassi comme principe actif, en association avec tout excipient approprié à l'administration par voie orale ou parentérale.

Les formes usuelles appropriées à l'administration orale ou parentérale peuvent être utilisées, telles que comprimés, dragées, gélules, capsules, cachets, solutés ou suspensions buvables ou solutés injectables.

## Revendications

1. Médicament, caractérisé en ce qu'il contient une fraction active extraite des rhizomes de Mandassi (*Cyperus articulatus* L.).

2. Composition pharmaceutique, caractérisée en ce qu'elle contient une fraction active extraite des rhizomes de Mandassi (*Cyperus articulatus* L.) en association avec tout excipient approprié.

3. Composition pharmaceutique selon la revendication 2, caractérisée en ce qu'elle est utile pour le traitement de l'épilepsie.

4. Procédé d'extraction d'une fraction de Mandassi (*Cyperus articulatus* L.), active comme anticonvulsivant, caractérisé en ce que l'on épuise des rhizomes de Mandassi broyés avec de l'eau, puis on concentre le soluté dans lequel on verse de l'alcool à 95° GL et on isole le précipité que l'on purifie et sèche.

## Claims

1. A medicament, characterised in that it contains a therapeutically-active fraction extracted from rhizomes of Mandassi (*Cyperus articulatus* L.).

2. A pharmaceutical composition, characterised in that it contains a therapeutically-active fraction extracted from rhizomes of Mandassi (*Cyperus articulatus* L.) in association with any suitable pharmaceutically acceptable excipient.

3. A pharmaceutical composition according to Claim 2, characterised in that it is useful for the treatment of epilepsy.

4. A process for the extraction of a fraction of Mandassi (*Cyperus articulatus* L.) which is active as an anticonvulsivant, characterised in that ground Mandassi rhizomes are extracted with water, the solution obtained is concentrated, 95° GL strength alcohol is poured into the concentrate, and the precipitate formed is isolated, purified and dried.

## Patentansprüche

1. Arzneimittel, dadurch gekennzeichnet, dass es eine aus Wurzelstöcken von Mandassi (*Cyperus articulatus* L.) ausgezogene Fraktion enthält.

2. Pharmazeutische Zubereitung, dadurch gekennzeichnet, dass sie eine wirksame, aus Wurzelstöcken von Mandassi (*Cyperus articulatus* L.) ausgezogene Fraktion enthält, in Kombination mit jedem geeigneten Hilfsstoff.

3. Pharmazeutische Zubereitung gemäss Anspruch 2, dadurch gekennzeichnet, dass sie zur Behandlung der Epilepsie nützlich ist.

4. Ausziehverfahren einer als Antikonvulsiva wirksamen Fraktion von Mandassi (*Cyperus articulatus* L.), dadurch gekennzeichnet, dass man zerkleinerte Mandassiwurzelstöcke mit Wasser erschöpft, dann die Lösung konzentriert, 95° GL Alkohol zugiesst und den Niederschlag isoliert, reinigt und trocknet.